# EUROPEAN PATENT APPLICATION

(11) **EP 4 755 865 A1**
(43) Date of publication of application: **10.06.2026**
(21) Application number: 25219893.2
(22) Date of filing: 01.12.2025
(51) Int. Cl.: C05F 11/10, A01G 33/00, A01N 63/20, A01N 65/03, A01P 21/00, C05G 3/80, C12N 1/12

(54) **BIOSTIMULANTS AND ENHANCEMENT THEREOF IN SPIRULINA**

(30) Priority: 05.12.2024 US 202463728171 P
(71) Applicant: Vaxa Technologies Ltd, 1200000 Rosh Pina (IL)
(72) Inventor: BASHAN, Ohad, 4493500 23 HaHadarim Street, Sde Varburg (IL); BERZIN, Isaac, 9350201 1 Ehud Street, Jerusalem (IL)
(74) Representative: Pearl Cohen UK

(57) **Abstract**

Biostimulant compositions and methods for enhancing soil organic carbon are provided. A mixture of bioavailable B12 and amino acids phenylalanine (Phe) and lysine (Lys) - was found to enhance soil organic carbon and crop growth due to synergistic effects between the bioavailable B12 and the PheLys. The bioavailable B12 and the PheLys may be derived from various sources, including, e.g., cyanobacteria (e.g., spirulina *- Arthrospira platensis*) or green algae (e.g., *Nannochloropsis spp.*) cultivated under photosynthetically controlled conditions to yield upregulated bioactive compounds such as bioavailable B12 and amino acids. One or more sources may be used to provide the bioavailable B12, Phe and Lys, and algal cultivation may be integrated with additional industries to yield synergistic biostimulation effects.

## Description

### BACKGROUND OF THE INVENTION

### 1. TECHNICAL FIELD

The present invention relates to the field of biostimulants for enhanced soil organic carbon and, more particularly, to biostimulants for enhanced soil organic carbon that provide a synergistic effect between amino acids and active vitamin B12.

### 2. DISCUSSION OF RELATED ART

Plant biostimulants are bioactive mixtures containing organic and inorganic materials that, when exogenously applied to a plant, e.g., via the soil, improves growth, vigor, productivity, nutrient uptake efficiency, and overall crop quality. In the last decades, the importance of amino acids in plant development and stress defense has become increasingly evident, attracting growing interest in basic and applied plant science. Besides being building blocks for protein synthesis, many amino acids, including some which are not involved in protein synthesis, turned out to have active roles in plant development and to participate in the plant's response to environmental stresses. In addition, amino acids serve as precursors for many primary and secondary metabolites and have pivotal roles in human nutrition, either as a source of nutraceutical compounds or as essential dietary components.

Lysine (Lys), as one of those essential amino acids, is often present at low levels in plants and thus limits their nutritional value. Yang et al. 2020 (Connections between amino acid metabolisms in plants: Lysine as an example. Frontiers in Plant Science 11:928) focused on lysine catabolism and described the connections between the degradation intermediates of this amino acid and other metabolic pathways, such as tryptophan metabolism, tricarboxylic acid cycle, and abiotic stress responses, starch metabolism, and the unfolded protein response. A closer look at the relationships between lysine and serine is provided by Kavi Kishor et al. 2015 (Role of proline in cell wall synthesis and plant development and its implications in plant ontogeny. Frontiers in Plant Science 6:544), who summarize the current knowledge on the biosynthetic pathways, regulatory mechanisms, and biological effects of both amino acids, focusing on the complexity of their interactions. The authors report the complex mechanisms of transcriptional and post-transcriptional regulation and highlight the importance of proteins rich in lysine and serine for plant development and stress tolerance.

While the interest in lysine biosynthesis largely aims at improving the nutritional value of crops, the research on the catabolism of this amino acid focuses more on tolerance to abiotic stresses. Lysine metabolism is involved in the plant's stress response in various forms. It is mainly catabolized through the saccharopine pathway, which has been shown to play a role in abiotic responses (see, e.g., Brown and Saa 2015, Biostimulants in agriculture, Frontiers in Plant Science 6:671). Moreover, under salt and osmotic stress, LKR/SDH expression was found to increase, while downstream metabolite pipecolate was enhanced (see, e.g., Nephali et al. 2020, Biostimulants for plant growth and mitigation of abiotic stresses: a metabolomics perspective, Metabolites 10(12):505). Our knowledge of amino acid metabolism has increased exponentially in the past three decades. Meanwhile, metabolism is one of the most important and complex networks within biological systems, yet our understanding of metabolic regulation remains limited in terms of the modular operation of these networks. Precise and detailed information on biological and molecular mechanisms and metabolic connections is therefore essential. The recent development of omics approaches has been widely applied to studies of amino acid metabolisms and their connections (see, e.g., Sun et al. 2024, Amino acids biostimulants and protein hydrolysates in agricultural sciences, Plants 13(2):210).

Many studies have reported the important and notable effects of the foliar application of Phenylalanine (Phe) on plants to overcome drought stress and increase total chlorophyll contents, shoot length, and biological yield. For example, Roman et al. 2020 (Foliar application of phenylalanine plus methyl jasmonate as a tool to improve Grenache grape aromatic composition. Sci. Hortic. 2020, 272, 109515) reported that foliar application of phenylalanine can increase the content of volatile compounds in grapes, and Portu et al. 2017 (Phenolic composition of Tempranillo grapes following foliar applications of phenylalanine and urea: A two-year study, Scientia Horticulturae 219: 191-199) introduced it as an important management tool for boosting grape quality.

Both Phnylalanine (Phe) and Lysin (Lys) are recognized as biostimulants in the EU and have the status of Annex IV REACH exemptions from the obligation to register in accordance with EU article 2(7)(a).

B12 biostimulant potential in combination humic acid (an organic compound naturally formed during long-term decomposition biomass residues) was demonstrated by Saadat et al. 2023 (Phenology, nitrogen status, and yield of red clover (Trifolium pretense L.) affected by application of vitamin B12, humic acid, and enriched biochar, Agronomy 2023, 13(12), 2885) - indicating that co-application of B12 and humic acid resulted in a boost in the root dry weight by 60% (3.8 to 6.4 g) while having no significant influence on the shoot dry weight of red clover (*Trifolium pretense* L.).

Nevertheless, B12 potential biostimulant activity, in concert with other biostimulants, was tested on horseradish roots (Rehim et al. 2021, Yield enhancement of biostimulants, vitamin B12, and CoQ10 compared to inorganic fertilizer in radish, Agronomy 11(4), 697) - yet no evidence was found to support vitamin B12 benefits as a biostimulant.

The physiologically active, bioavailable forms of B12, termed cobalamins, have a 5,6-dimethylbenzimidazole (DMBI) as the lower axial ligand coordinated to the central cobalt ion, such as in methylcobalamin and 5'-deoxyadenosylcobalamin. In contrast, non-active pseudo forms of B12, termed cobamides have a different base (e.g., adenine) substituting for DMBI and are not bioavailable. It is noted that cyanocobalamin is a synthetic cobalamin that includes the DMBI base, but is not active due to its cyano group, which may be replaced physiologically with a methyl group or a 5'-deoxyadenosyl group to form the two active forms.

Commercial algal extracts are typically made of cyanobacteria (e.g., spirulina - *Arthrospira platensis*) or green algae (e.g., *Nannochloropsis spp*.) grown in open ponds and/or other solar-based cultivation systems. Commercially grown algae (e.g., spirulina) typically comprise pseudo-B12 (also termed cobamide, in which the lower ligand of the molecule (5,6-dimethylbenzimidazole (DMB)) is replaced by adenine as a base), which opposes and reduces its bioavailability. Pseudo-vitamin B12 typically refers to compounds that are corrinoids with a structure similar to vitamin B12 but without vitamin activity, and is therefore metabolically unavailable. For example, van der Oever and Mayer 2022 (Biologically active or just "pseudo"-vitamin B12 as predominant form in algae-based nutritional supplements?, Journal of Food Composition and Analysis, 10, 104464), incorporated herein in their in its entirety, teaches a simple and fast ultra-high performance liquid chromatography method with UV detection to measure algae-based nutritional supplements contain both physiologically active vitamin B12 (e.g., cobalamin in various forms, such as methyl-, cyano-, hydroxo- and adenosyl-cobalamin) and its non-active pseudo-form. The measurements show a big variation in the concentration of pseudo-vitamin B12 within all samples analyzed.

Spirulina algae (*Arthrospira platensis*) cultivated in geothermally powered photobioreactors were proposed as a potentially resource efficient, zero-carbon, and nutritious alternative to conventional beef meat, e.g., in Tzachor et al. 2022 (Environmental Impacts of Large-Scale Spirulina (Arthrospira platensis) Production in Hellisheidi Geothermal Park Iceland: Life Cycle Assessment, Marine Biotechnology 24:991-1001), incorporated herein in their in its entirety.

### SUMMARY OF THE INVENTION

The following is a simplified summary providing an initial understanding of the invention. The summary does not necessarily identify key elements nor limit the scope of the invention, but merely serves as an introduction to the following description.

One aspect of the present invention provides biostimulant compositions for enhancing soil organic carbon, the biostimulant compositions comprise bioavailable B12 and PheLys comprising phenylalanine (Phe) and lysine (Lys).

One aspect of the present invention provides methods of enhancing soil organic carbon, the methods comprise applying a mixture of bioavailable B12 and PheLys comprising phenylalanine (Phe).

These, additional, and/or other aspects and/or advantages of the present invention are set forth in the detailed description which follows, possibly inferable from the detailed description, and/or learnable by practice of the present invention.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:
**Figure 1A** illustrates the experimental setting of the plots and the respective treatment applied to each plot, and **Figure 1B** illustrates the results, indicating the synergistic effect of using disclosed extracts with both bioavailable B12 and Phe and Lys amino acids, according to some embodiments of the invention.
**Figures 1C** and **1D** are a high-level schematic illustration and a flowchart, respectively, of the synergistic integration of algal products with agriculture and various industries and methods thereof, according to some embodiments of the invention.
**Figures 2A-2F** provide chromatograms with experimental results that indicate the difference between pseudo-B12 and active methylcobalamin (MeB12) in the disclosed and prior art extracts.
**Figure 3A** is a high-level schematic flowchart illustrating methods of enhancing soil organic carbon, according to some embodiments of the invention.
**Figure 3B** is a high-level schematic flowchart illustrating cultivation, extraction and treatment methods, according to some embodiments of the invention.
**Figures 4A-4D** are high-level schematic illustrations of cultivation systems, according to some embodiments of the invention.

These, additional, and/or other aspects and/or advantages of the present invention are set forth in the detailed description which follows; possibly inferable from the detailed description; and/or learnable by practice of the present invention.

### DETAILED DESCRIPTION OF THE INVENTION

In the following description, various aspects of the present invention are described. For purposes of explanation, specific configurations and details are set forth in order to provide a thorough understanding of the present invention. However, it will also be apparent to one skilled in the art that the present invention may be practiced without the specific details presented herein. Furthermore, well known features may have been omitted or simplified in order not to obscure the present invention. With specific reference to the drawings, it is stressed that the particulars shown are by way of example and for purposes of illustrative discussion of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before at least one embodiment of the invention is explained in detail, it is to be understood that the invention is not limited in its application to the details of construction and the arrangement of the components set forth in the following description or illustrated in the drawings. The invention is applicable to other embodiments that may be practiced or carried out in various ways as well as to combinations of the disclosed embodiments. Also, it is to be understood that the phraseology and terminology employed herein are for the purpose of description and should not be regarded as limiting.

Some embodiments of the present invention provide efficient and economical methods and biostimulant compositions for enhancing soil organic carbon, and thereby provide improvements to the technological field of agriculture. Biostimulant compositions and methods for enhancing soil organic carbon are provided. A mixture of bioavailable B12 and amino acids phenylalanine (Phe) and lysine (Lys) - was found to enhance soil organic carbon and crop growth due to synergistic effects between the bioavailable B12 and the PheLys. The bioavailable B12 and the PheLys may be derived from various sources, including, e.g., cyanobacteria (e.g., spirulina *- Arthrospira platensis*) or green algae (e.g., *Nannochloropsis spp*.) cultivated under photosynthetically controlled conditions to yield upregulated bio-active compounds, such as bioavailable B12 and amino acids. One or more sources may be used to provide the bioavailable B12, Phe and Lys, and algal cultivation may be integrated with additional industries to yield synergistic biostimulation effects.

Disclosed experiments demonstrate the biostimulating effect of applying an amino acid mix of Phenylalanine and Lysine (PheLys) together with active, bioavailable vitamin B12. The biostimulating effect was demonstrated, in a non-limiting manner, on the yield of carrot roots (*Daucus carota* L. subsp. *sativus* Thell.). Carrot as a model crop has a root system with relatively large mass, and is used as indication for the soil organic carbon (SOC) composition, as the carrot roots are a major component of the SOC.

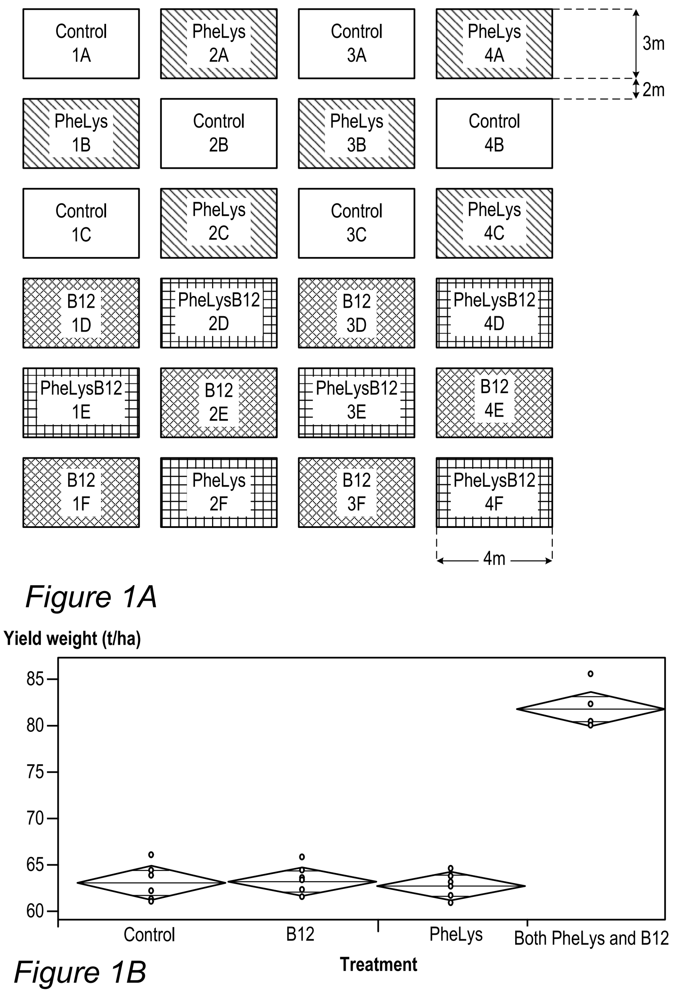

**Figure 1A** illustrates the experimental setting of the plots and the respective treatment applied to each plot. Distances between plots are indicated in meters. **Table 1** provides the results, and **Figure 1B** illustrates the data from **Table 1** in graph form, indicting the synergistic effect of using disclosed extracts with both bioavailable B12 and Phe and Lys amino acids, according to some embodiments of the invention - compared with using either treatment by itself.

The experimental setting included carrot cultivation in detached light sandy loam soil plots that provide soil conditions (with respect to soil texture and chemical properties) in which carrots thrive. A soil chemical analysis was performed to verify the chemical properties, resulting in the following chemical properties: Organic matter - 1.23 %wt., pH=6.4, NO₃ content - 9.6 mg/l, NH₄ content - 30.1 mg/l, P₂O₅ content 166.0 mg/l, K₂O content 115.7 mg/l, and Mg content 76.6 mg/l. All plots were grown under the same conditions (soil conditions, weather, irrigation of 3,000 1·ha⁻¹) and were split into four groups as illustrated schematically in **Figure 1A**. Heirloom carrot seeds were planted on ridges 67.5 cm apart from each other. Seeding at a rate of 3.5 kg·ha-1 was completed with a hand-held wheelbarrow vegetable drill, with two rows per ridge. Each of the four groups included six plots, each measuring 12 m² (3 m x 4 m). The carrots were harvested from the central 6 m² of each plot, to avoid edge effects, and path of 2 m was left between experimental lots to ensure isolation between the plots with respect to the treatments.

In the plots of the control group no further treatment was applied.

In the plots of the B12 group, disclosed bioavailable B12 extracted form spirulina (*Arthrospiras platensis,* UTEX LB1926) grown under the disclosed conditions, involving intense illumination, mixing and CO₂ bubbling, was applied as soil treatment. It is noted that similar results are expected independently of the source of the bioavailable B12, which may also be derived from other algae grown under various conditions, as well as from yeast and bacteria. B12 dry powder was dissolved in water to yield a 0.3 mg/l B12 water solution, which was included in the same total amount of irrigation, applied to the leaves and the soil, resulting in an application rate of 2 mg/ha of vitamin B12 at a stage of 4-6 carrot leaves, or BBCH scale 14-16.

**In** the plots of the PheLys group, phenylalanine and lysine were applied as soil treatment. PheLys dry powder including Phe 3.22wt% and Lys 3.92wt% was used to prepare a 1%wt water solution, in which the amino acids were dissolved, which was included in the same total amount of irrigation, applied to the leaves and the soil, resulting in an application rate of 67.5 g/ha of Phe and Lys at a stage of 4-6 carrot leaves, or BBCH scale 14-16. The amino acid dosage used was as proposed, e.g., by Mustafa et al. 2016, Response of okra (Abelmoschus esculentus L.) to soil and foliar applied L-tryptophan. Soil Environ. 35, 76-84, Rosa et al., 2023, Effect of L-Tryptophan and L-Glutamic acid on carrot yield and its quality, Agronomy 13, 562; and Mazher et al. 2011, Stimulatory effect of kinetin, ascorbic acid and glutamic acid on growth and chemical constituents of Codiaeum variegatum L. Plants, American-Eurasian J. Agric. & Environ. Sci., 10 (3): 318-323.

Finally, in the plots of the PheLys and B12 group, a combination of the PheLys and B12 treatments was applied to the plots. PheLys and B12 dry powders were used to prepare the water solution by dissolving the amino acids in water (10g/l) and adding the bioavailable B12 dry powder (0.3 mg/l). The water solution, in which the amino acids and B12 were dissolved was included in the same total amount of irrigation, applied to the leaves and the soil, resulting in an application rate of 67.5 g/ha of Phe and Lys and 2 mg/ha of B12 at the stage of 4-6 carrot leaves, or BBCH scale 14-16.

The results **(Table 1,** **Figure 1B****)** clearly indicate a strong and statistically significant (t-test p-value <0.0001) synergetic effect between the application of PheLys and bioavailable B12, which was not present for the application of either PheLys or B12 alone. While the results from the PheLys plots and from the B12 plots are similar to the untreated control plots, the synergetic effect of PheLys and bioavailable B12 increased the yield by ca. 30% - indicating a strong biostimulatory effect. It is noted that in all groups, the ratio of dry to wet weight was found to be similar (5.4±0.7%) - indicating that the synergistic effect relates directly to added biological (carbon) material, and not merely to added water.

**Table 1: Results concerning yields in the plots illustrated in Figure 1A, with respect to the applied treatment. The yields are on a wet weight (WW) basis, in tons per hectare.**

| | Control | | B12 | | PheLys | | PheLys and B12 | |
|---|---|---|---|---|---|---|---|---|
| | Plot | Yield | Plot | Yield | Plot | Yield | Plot | Yield |
| Data | 1a | 61.1 | 1d | 61.6 | 2a | 61.8 | 1d | 82.2 |
| | 1c | 62.1 | 3d | 63.3 | 4a | 62.8 | 4d | 80.3 |
| | 2b | 64.4 | 2e | 63.4 | 1b | 64.4 | 1e | 85.4 |
| | 3a | 63.8 | 4e | 64.2 | 3b | 63.4 | 3e | 80.0 |
| | 4b | 66.1 | 1f | 66.0 | 2c | 63.9 | 2f | 80.1 |
| | 3c | 61.3 | 3f | 62.1 | 4c | 61.3 | 4f | 82.2 |
| Average | 63.1 t/ha | | 63.4 t/ha | | 62.9 t/ha | | 81.7 t/ha | |
| STDV | 1.8 t/ha | | 1.4 t/ha | | 1.1 t/ha | | 1.9 t/ha | |

In accordance with the experimental results, various embodiments comprise biostimulant compositions for enhancing soil organic carbon, and their uses to enhance soil organic carbon and increase growth of various crops. The biostimulant compositions comprise bioavailable B12 and PheLys comprising phenylalanine (Phe) and lysine (Lys).

In various embodiments, the bioavailable B12 and/or the PheLys may be used in dry powder form and/or may be mixed in water to form a biostimulant solution. In non-limiting examples, in the biostimulant solution, bioavailable B12 may be between 0.2-0.5 mg/l, between 0.1-10 g/l, or have intermediate values; and PheLys may be between 5-15 g/l, between 2-50 g/l, or have intermediate values. In non-limiting examples, the biostimulant compositions may have a ratio between bioavailable B12 and PheLys that yields an application of between 50-80 g/ha, between 10-200 g/ha or having intermediate values of Phe and Lys; and between 1-5 mg/ha, between 0.1-50 mg/ha or having intermediate values of bioavailable B12. It is noted that the exact concentrations and ratios depend on various parameters, such as the type of the crop, the soil conditions, and various growth conditions.

In some embodiments, the bioavailable B12 and/or Phe/Lys may be extracted from spirulina, including *Arthrospira spp.,* cultivated under photosynthetically controlled conditions to yield upregulated bio-active compounds comprising bioavailable B12 that is more bioavailable than pseudo-B12 and/or increased content of PheLys. The photosynthetically controlled conditions may comprise cultivating the *Arthrospira spp.* under 31±2°C, at pH of 10.8 ± 0.2 and under irradiance of between 700 and 1500 µmol/(m²s) and/or cultivating the *Arthrospira spp.* in a ultra-high-density culture having a density between 3g/l and 10g/l or between 5g/l and 15g/l and under ultraviolet radiation intensity of between 70-150 µmol/m²s.

In some embodiments, the bioavailable B12 and/or the PheLys may be extracted from *Nannochloropsis spp.* cultivated under photosynthetically controlled conditions to yield upregulated bio-active compounds comprising bioavailable B12 that is more bioavailable than pseudo-B12 and/or increased content of PheLys. The photosynthetically controlled conditions may comprise cultivating the *Nannochloropsis spp.* under 15-25°C, at pH of 10.8 ± 0.2 and under irradiance of between 700 and 1500 µmol/(m²s) or under solar irradiance and/or cultivating the *Nannochloropsis spp.* in a ultra-high-density culture having a density between 3g/l and 10g/l or between 5g/l and 15g/l and under ultraviolet radiation intensity of between 70-150 µmol/m²s.

The bioavailable B12 and/or the PheLys may be made of a water-based extract that is produced by water-extraction and cycles of freezing and thawing applied to the cultivated algae, e.g., cyanobacteria *Arthrospira spp.* and/or green algae *Nannochloropsis spp.* In some embodiments, the bioavailable B12 and/or the PheLys may comprise fractionated compounds of the water-based extract.

The bioavailable B12 and/or the PheLys may be made of the algal biomass and/or wet/dry extracts thereof, and are characterized by high levels of bioavailable B12 (e.g., methylcobalamin, 5-deoxyadenosylcobalamin or other metabolically active forms of B12) and/or Phe/Lys, providing more bioavailable B12 than pseudo-B12 and/or increased content of PheLys. The spirulina grown under disclosed conditions may have more bioavailable B12 than pseudo-B12 (e.g., at least two or three times more bioavailable B12 than pseudo-B12) - providing bioavailable B12 to enhance soil organic carbon content.

The bioavailable B12 and/or the PheLys may be prepared from any of the water-based algal extract without further processing, dried extracts, algal (e.g., *spirulina*/*Nannochloropsis)* biomass, dried biomass, algal (e.g., spirulina/*Nannochloropsis*) paste, algal (e.g., *spirulina*/*Nannochloropsis)* powder and/or algal (e.g., *spirulina*/*Nannochloropsis)* liquid. Water-based extracts may be prepared by water extraction of algal (e.g., *spirulina*/*Nannochloropsis)* biomass that is cultivated under controlled, ultra-high-density conditions with strong UV illumination and strong continuous mixing, and are characterized by high levels of bioavailable B12 (e.g., methylcobalamin, 5-deoxyadenosylcobalamin or other metabolically active forms of B12), as contrasted with prior art B12 products that include large amounts of pseudo-B12, as well as increased content of PheLys. Water-based algal (e.g., *spirulina*/*Nannochloropsis)* extracts may be produced by water-extraction and cycles of freezing and thawing applied to the cultivated algae (e.g., *spirulina*/*Nannochloropsis).*

In some embodiments, the bioavailable B12 and/or the PheLys may be derived from cultivated yeast and/or cultivated bacteria. In various embodiments, waste products of animal cultivation, cement industry and/or packaging industry may be further used to provide biostimulants as disclosed herein.

Further analysis of the proteins in the spirulina powders yielded the following concentrations of amino acids (constituting 87.8% of the total protein content): Aspartic acid 6.50 g/100g, Threonine 3.76 g/100g, Serine 3.44 g/100g, Glutamic acid 17.54 g/100g, Proline 2.72 g/100g, Glycine 3.74 g/100g, Alanine 7.60 g/100g, Valine 4.56 g/100g, Methionine 1.57 g/100g, Isoleucine 3.82 g/100g, Leucine 6.01 g/100g, Tyrosine 2.98 g/100g, Phenylalanine 3.22 g/100g, Histidine 1.15 g/100g, Lysine 3.92 g/100g and Arginine 4.52 g/100g.

In an analysis of the amino acids content of *Nannochloropsis* algae grown under controlled conditions, the following results were found (with respect to 100wt% protein basis): Essential amino acids: Arginine - 6wt%, Histidine - 2wt%, Isoleucine - 4wt%, Leucine - 9wt%, Lysine - 8wt%, Methionine - 2wt%, Phenylalanine - 5wt%, Threonine - 5wt%, Valine - 6wt%, and non-essential amino acids: Alanine - 3wt%, Aspartic acid - 11wt%, Cystine - 1wt%, Glutamic acid - 10wt%, Glycine - 6wt%, Hydroxyproline <1wt%, Proline - 6wt%, Serine - 4wt%, Tyrosine - 3wt%.

In various embodiments, Phe and Lys in the algal extract may range between 3-5wt% and 4-8wt%, respectively. Under intensive illumination and high density growing conditions disclosed herein, even higher concentrations of Phe (Phenylalanine) and Lys (Lysine were achieved), namely 12.0 - 12.3 g Phe per 100 g dry biomass and 13.9 - 14.3 g Lys per 100 g dry biomass, in addition to the higher content of bioavailable bioavailable B12 compared to pseudo-B12 described, e.g., by Tzachor et al. 2024 (Photonic management of Spirulina (Arthrospira platensis) in scalable photobioreactors to achieve biologically active bioavailable vitamin B12, Discover Food 4, 69), carried out in cooperation with the inventors and using disclosed cultivation methods at the Hellisheidi Spirulina facility in Hellisheiði (Hellisheidi) geothermal power park, in the region of Hengill, Iceland - and incorporated herein incorporated herein in its entirety. Accordingly, disclosed cultivation conditions reaches a PheLys concentration of 26.6 g PheLys per 100 g dry biomass - providing a large increase compared to prior art cultivation under solar irradiation of 2.8-2.9 g Phe per 100 g dry biomass and 2.9-3.4 g Lys per 100 g dry biomass reported in the literature, resulting in a mere prior art PheLys concentration of 5.7-6.3 g PheLys per 100 g dry biomass (highest prior art data in Masten Rutar et al. 2022, Nutritional quality and safety of the spirulina dietary supplements sold on the Slovenian market. Foods 2022, 11, 849). Hence, disclosed cultivation systems and methods provide a 4-5 fold increase in the content of PheLys in spirulina, in addition to the unprecedented reversal of the quantitative relations between pseudo B12 and bioavailable B12 (see Sabrina *et al.* 2022).

Some embodiments include a dry mass extract of cultivated *Arthrospira spp.* that includes more than 10 g Phe per 100 g dry biomass, more than 10 g Lys per 100 g dry biomass and at least twice bioavailable B12 compared pseudo B12.

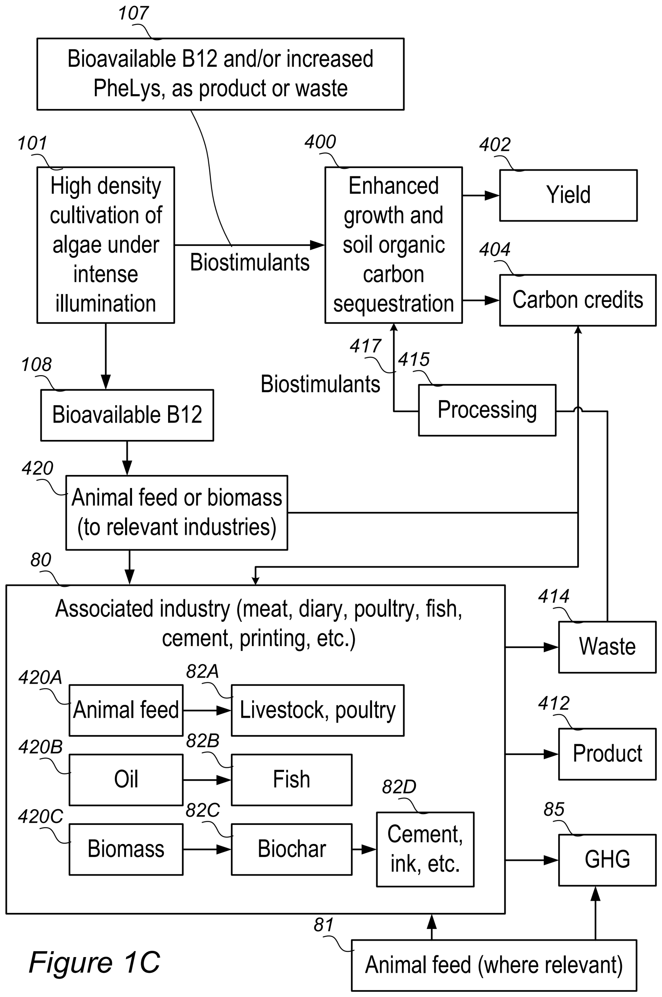

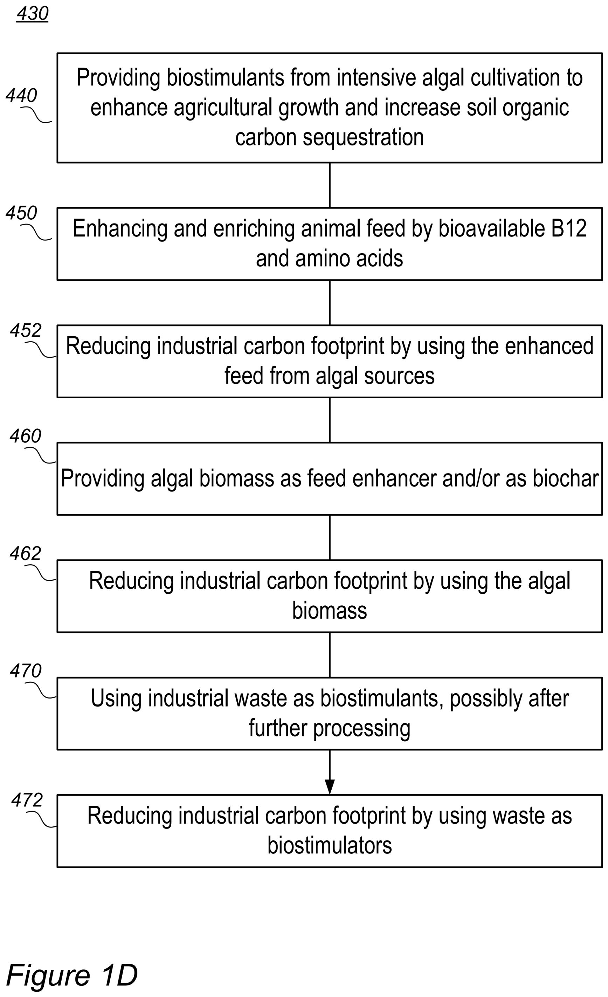

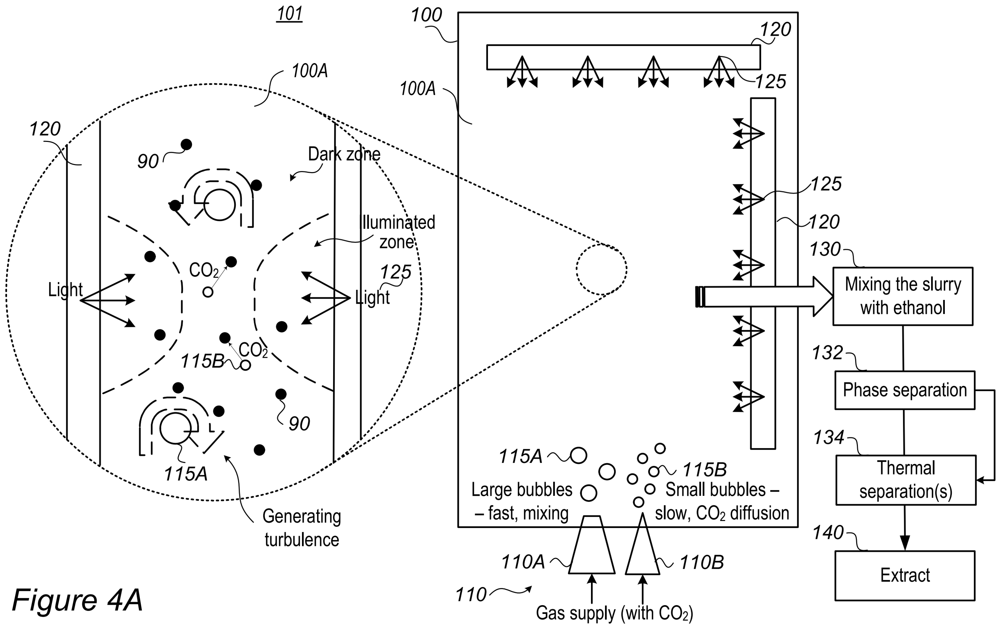

**Figures 1C** and **1D** are a high-level schematic illustration and a flowchart, respectively, of the synergistic integration of algal products with agriculture and various industries and methods thereof, according to some embodiments of the invention. High density cultivation of algae under intense illumination **101,** as disclosed herein (see, e.g., **Figures 4A-4D**) provides biostimulants **107** such as disclosed compositions of bioavailable B12 (in large excess of pseudo B12) and PheLys at increased quantities to enhance agricultural growth and increase soil organic carbon sequestration **400** (see experimental results in **Figure 1B**). In addition, algal biomass may be used as feed enhancer **420** for various animal growing operations **80** (e.g., of livestock, poultry etc. **82A,** fish **82B,** etc.) providing bioavailable B12 **108** and additional nutrients (e.g., enhanced content of amino acids) in animal feed **420A** and/or specific fractions such as enriched oil **420B** (e.g., having high content of omega 3 and omega 7 fatty acids - see, e.g., U.S. Patent Application Publication No. 20230014004, incorporated herein by reference in its entirety). Enhanced agricultural growth and increase soil organic carbon sequestration **400** thus provide larger crops (yield **402**) as well as carbon credits **404** due to the increased carbon content of the crops, demonstrated herein. Similarly, algal biomass **420C** may be used to produce biochar **82C** as a carbon source for additional industries **80** such as the cement industry and the printing industry **82D,** e.g., providing ink for printing packages. All these industries **80** are characterized by production of products **412** (e.g., meat, dairy products, eggs, fish, cement, printed goods, etc.), waste **414** and large greenhouse gas (GHG) emissions **85.**

Moreover, synergy may be achieved by using waste **414** from associated industries **80** to further enhance growth and increase soil organic carbon sequestration **400,** utilizing organic material in wastes **414,** possibly following further processing **415** - as additional biostimulants **417.** Biostimulation form any of direct algal products (bioavailable B12 and/or increased PheLys) and/or wastes of algal production **107** (e.g., in case bioavailable B12 and increased amino acid content are utilized for other purposes, such as animal feed **420** described above) and/or waste **414** of associated industries - may thus be used to increase yield **402** and to receive carbon credits **404** due to the increased carbon content of the crops. It is noted that carbon credits **404** may also be received due to the animal feed or biomass **420** which are provided from cultivation facilities **101** having a negative carbon footprint (due to CO₂ sequestration in the process of growing the algae), and due to the reduction in the use of regular animal feed **81** (e.g., in livestock, poultry and fish industries) and associated GHG emissions - by replacing gat least part thereof with algal-based feed **420.** The gained carbon credits **404** may be used to reduce or balance GHG emissions **85** from the respective industries **80.** Additional synergic benefits, beyond enhanced agricultural growth and increase soil organic carbon sequestration **400** include broadening the scope of available arable land, as biostimulants enable use of degraded soils.

In some embodiments, disclosed synergy may even enable commercial production of biostimulants **417** from various types of waste **414** - as the carbon credits **404** may be used as an additional financial benefit of the process, offsetting high processing costs via enhanced agricultural growth and increase soil organic carbon sequestration **400.**

**Figure 1D** schematically illustrates method **430** comprising providing biostimulants from intensive algal cultivation to enhance agricultural growth and increase soil organic carbon sequestration (stage **440**). Methods **430** may further comprise enhancing and enriching animal feed by bioavailable B12 and amino acids (stage **450**) and reducing industrial carbon footprint by using the enhanced feed from algal sources (stage **452**).

Method **430** may further comprise providing algal biomass as feed enhancer and/or as biochar (stage **460**) and reducing industrial carbon footprint by using the algal biomass (stage **462**).

Method **430** may further comprise using industrial waste as biostimulants, possibly after further processing (stage **470**) and reducing industrial carbon footprint by using waste as biostimulators (stage **472**).

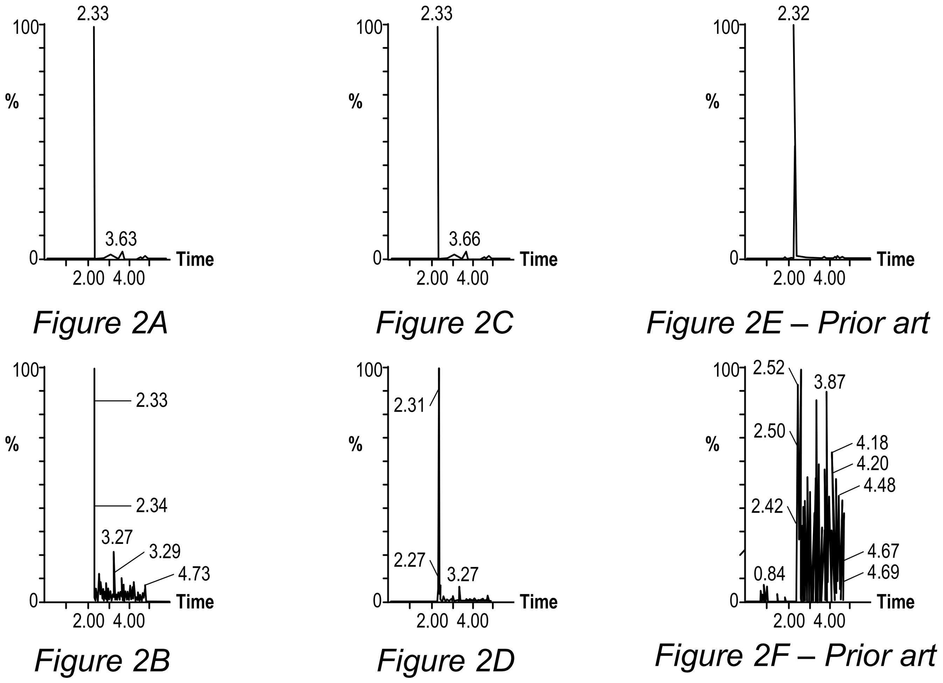

**Figures 2A-2F** provide chromatograms with experimental results that indicate the difference between pseudo-B12 and bioavailable MeB12 in the disclosed and prior art extracts. The results relate to spirulina, including *Arthrospira* spp. that was cultivated under photosynthetically controlled conditions to yield upregulated bio-active compounds comprising bioavailable B12 that is more bioavailable than pseudo-B12 and/or increased content of PheLys. It is noted that, while spirulina extracts are typically prepared from cyanobacteria such as *Arthrospira spp.,* the term algae (as in blue-green algae) is also commonly used to denote the cyanobacteria due to their photosynthetic capabilities and chlorophyll content. While in some of the results disclosed herein bioavailable, bioavailable B12 was measured as methylcobalamin (MeB12), disclosed extracts also include other forms of bioavailable, bioavailable B12, such as 5-deoxyadenosylcobalamin, which is another form of metabolically active vitamin B12 (contrasted with pseudo-B12 which are forms that are not metabolically available).

Specifically, **Figures 2A** and **2B** provide chromatograms for bioavailable MeB12 and pseudo-B12, respectively, in the water soluble (blue) fraction of the disclosed extracts; **Figures 2C** and **2D** provide chromatograms for bioavailable MeB12 and pseudo-B12, respectively, in the water insoluble (green) fraction of the disclosed extracts - both pairs of graphs indicating the purity of bioavailable MeB12 in the disclosed extracts, while **Figures 2E** and **2F** provide chromatograms for bioavailable MeB12 and pseudo-B12, respectively, in prior art extracts indicating the large extent of additional MeB12-like compounds termed pseudo-B12 (see the multiple peaks in **Figure 2F**), which are not bioavailable and even oppose the bioavailability of MeB12. As is evident in the data, disclosed extracts and fractions thereof yield MeB12 which is much more pure than prior art extracts/powders and commercial products from algae grown in open ponds, under different conditions than presently disclosed. The inventors suggest that the high purity results from the growing and extraction conditions of the disclosed extracts, and may be responsible for the high bioavailability of MeB12 in the disclosed extracts. For example, the inventors suggest that the controlled light conditions (spectral composition, intensity, photo-modulation) may activate different metabolic pathways in spirulina/*Arthrospira* cyanobacteria that may enable or enhance the synthesis of bioavailable methylcobalamin.

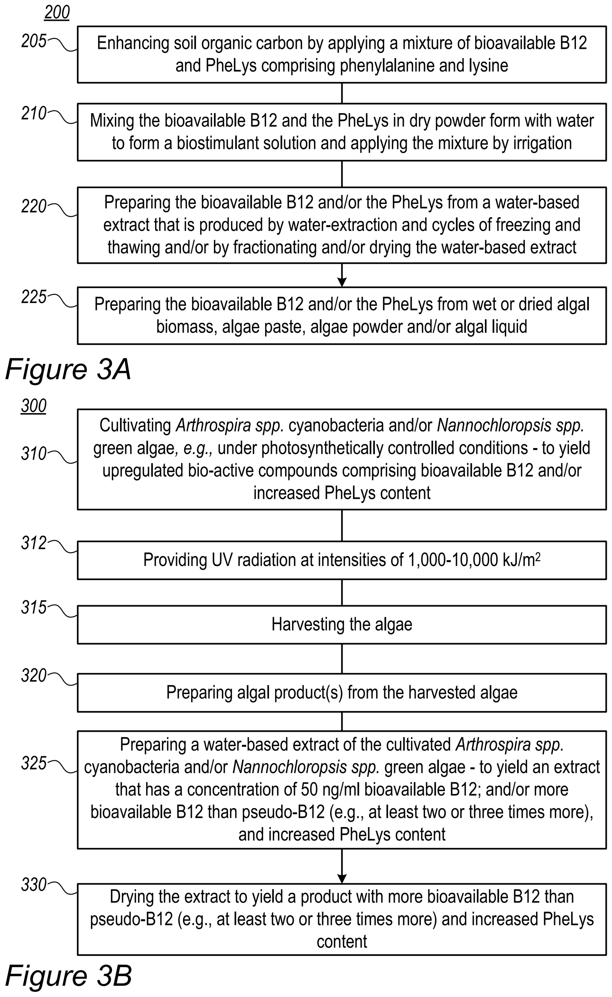

**Figure 3A** is a high-level schematic flowchart illustrating methods **200** of enhancing soil organic carbon, according to some embodiments of the invention. The method stages may be carried out with respect to the biostimulant compositions described herein. Method **200** may comprise the disclosed stages, irrespective of their order.

Method **200** comprises enhancing soil organic carbon by applying a mixture of bioavailable B12 and PheLys comprising phenylalanine (Phe) and lysine (Lys) (stage **205**). The bioavailable B12 and/or the PheLys may be derived from spirulina (e.g., a spirulina extract), made of *Arthrospira spp.* that was cultivated under photosynthetically controlled conditions to yield upregulated bio-active compounds comprising bioavailable B12 that is more bioavailable than pseudo-B12, and/or increased content of PheLys as disclosed herein. Alternatively or complementarily, the bioavailable B12 and/or the PheLys may be derived from *Nannochloropsis spp.* (e.g., from a *Nannochloropsis* extract), made of green algae cultivated under photosynthetically controlled conditions (or possibly under solar irradiance) to yield upregulated bio-active compounds comprising bioavailable B12 that is more bioavailable than pseudo-B12 and/or increased content of PheLys, as disclosed herein. In some embodiments, the bioavailable B12 and/or the PheLys may be derived from yeast and/or from bacteria.

Method **200** may further comprise mixing the bioavailable B12 and the PheLys in dry powder form with water to form a biostimulant solution and applying the mixture by irrigation (stage **210**).

In various non-limiting examples, in the biostimulant solution, bioavailable B12 may be between 0.2-0.5 mg/l and PheLys may be between 5-15 g/l. In some embodiments, the mixture may have a ratio between bioavailable B12 and PheLys that yields an application of between 50-80 g/ha of Phe and Lys and between 1-5 mg/ha of bioavailable B12.

As disclosed herein, bioavailable B12 and optionally PheLys may be derived from cyanobacteria (e.g., *Arthrospira spp*.) and/or from green algae (*Nannochloropsis spp*.) as well as optionally from yeast or bacteria.

For example, bioavailable B12 and optionally PheLys may be derived from spirulina (*Arthrospira spp*.) cultivated under photosynthetically controlled conditions that comprise 31±2°C, at pH of 10.8 ± 0.2 and under irradiance of between 700 and 1500 µmol/(m²s), and/or comprise a ultra-high-density culture having a density between 3g/l and 10g/l or between 5g/l and 15g/l and under ultraviolet radiation intensity of between 70-150 µmol/m²s.

In another example, bioavailable B12 and optionally PheLys may be derived from *Nannochloropsis spp.* cultivated under photosynthetically controlled conditions that comprise cultivating the *Nannochloropsis spp.* under 15-25°C, at pH of 10.8 ± 0.2 and under irradiance of between 700 and 1500 µmol/(m²s) or under solar irradiance and/or cultivating the *Nannochloropsis spp.* in a ultra-high-density culture having a density between 3g/l and 10g/l or between 5g/l and 15g/l and under ultraviolet radiation intensity of between 70-150 µmol/m²s.

In some embodiments, method **200** may further comprise preparing the bioavailable B12 and/or the PheLys from a water-based extract that is produced by water-extraction and cycles of freezing and thawing applied to the cultivated algae (e.g., cyanobacteria such as *Arthrospira spp.* and/or green algae such as *Nannochloropsis spp.)* and/or preparing the bioavailable B12 and/or the PheLys by fractionating and/or drying the water-based extract (stage **220**).

In some embodiments, method **200** may further comprise preparing the bioavailable B12 and/or the PheLys from at least one of: the wet or dried algal biomass, algae paste made of the algal biomass, algae powder and/or algal liquid made of the algal biomass (stage **225**).

**Figure 3B** is a high-level schematic flowchart illustrating cultivation and extraction methods **300,** according to some embodiments of the invention. The method stages may be carried out with respect to cultivation system **101** described herein, which may optionally be configured to implement method **300.** Method **300** may be at least partially implemented by at least one computer processor, e.g., in a controller **103** comprising corresponding processing unit(s). Certain embodiments comprise computer program products comprising a computer readable storage medium having computer readable program embodied therewith and configured to carry out the relevant stages of method **300.** Method **300** may comprise the disclosed stages, irrespective of their order.

As illustrated schematically in **Figure 3B****,** method **300** of preparing an algal product may comprise cultivating *Arthrospira spp.* cyanobacteria and/or *Nannochloropsis spp.* green algae under photosynthetically controlled conditions to yield upregulated bio-active compounds comprising bioavailable methylcobalamin B12 (MeB12) (stage **310**), e.g., in cultivation system 101. Illumination may be provided by at least one of light sources **125,** configured to emit UV light at both UVA and UVB spectra. In some embodiments, the ratio between the emitted intensities of UVA/UVB radiation may be in a range of 10-15, for example, 10 UBA/UVB. In certain embodiments, method **300** may be used to grow cyanobacteria, e.g., *Arthrospira spp.* and/or green algae, e.g., *Nannochloropsis spp.,* from which algal products are produced. Method **300** may further comprise providing the UV radiation at intensities of 1,000-10,000 kJ/m² (stage **312**), for example, 5000 kJ/m² or at any other intermediate value. For example, controller **103** may be configured to control the provision of the UV radiation using on/off radiation pulses. In some embodiments, each pulse may last 0.0099 sec, and between 1-100 of such pulses may be provided per second, for example, 10 times per second. It is noted that approximately 0.01sec of 1,000kJ/m² illumination yields about ten times the intensity of solar UV radiation, which changes the chemical composition of the algae and/or cyanobacteria to yield products, extracts and compositions disclosed herein.

Method **300** further comprises harvesting the algae (cyanobacteria, blue-green algae, or green algae) (stage **315**), e.g., implementing continuous harvesting and matching the harvest rate to the growth rate. Method **300** further comprises preparing algal product(s) from the harvested algae (stage **320**), e.g., from the algal biomass and/or from wet or dry extracts thereof. In various embodiments, algal products may be prepared from at least one of: the water-based extract without further processing, dried extracts, algal biomass, dried biomass, algae paste, algae powder and/or algal liquid.

For example, method **300** may comprise preparing a water-based extract of the cultivated *Arthrospira spp.* cyanobacteria and/or *Nannochloropsis spp.* green algae to yield an algal extract that has a concentration of at least 50 ng/ml bioavailable B12 and/or more bioavailable B12 than pseudo-B12 (stage **325**), e.g., at least two or three times more. Bioavailable, or bioavailable B12 refers to the metabolically active forms of vitamin B12 such as methylcobalamin and 5-deoxyadenosylcobalamin, in contrast to pseudo-B 12 which represents metabolically inactive forms.

The extraction may be carried out by applying one or more freeze-thaw cycles to break the cell walls and enhance the extractability of the suspension. For example, the harvested biomass may be rapidly frozen to -20°C then thawed at 0 to 4 °C until completely de-frosted. Method **300** may further comprise drying the extract to yield an algae powder having more bioavailable B12 than pseudo-B12 (stage **330**), e.g., at least two or three times more. Products, biomass, extracts (wet or dry) or derivatives thereof may be prepared as described herein.

In certain embodiments, a wet biomass of spirulina may be suspended in pure water (hot or cold) to obtain a product with 10 weight % dry substance. The insoluble substances may be removed by continuous centrifugation. The supernatant, containing soluble biologically active substances, may be used as botanical extracts, which may comprise water-soluble pigments (e.g., phycocyanin), proteins. nucleic acids, polysaccharides and ash. These compounds may be further fractionated (e.g., by chromatography and ethanol precipitation) in order to enhance the content of bioavailable (e.g., bioavailable) B12.

Various embodiments comprise combinations of steps from methods **200, 300** and **430.**

**Figures 4A-4D** are high-level schematic illustrations of cultivation systems **101,** according to some embodiments of the invention. Cultivation system **101** is configured to grow algae and/or cyanobacteria in one or more bioreactor(s) **100,** at high density and under high illumination intensity. Elements from **Figures 4A-4D** may be combined in any operable combination, and the illustration of certain elements in certain figures and not in others serves merely an explanatory purpose and is non-limiting. It is noted that any disclosed value may be modified by ±10% of the value.

**Figure 4A** is a high-level schematic illustration of a cultivation systems **101** with bioreactor(s) **100** and extraction equipment for extracting botanical extracts **140,** according to some embodiments of the invention. Bioreactor **101** comprises one or more tanks filled with a growth medium **100A** (e.g., water, optionally with additives) and *Arthrospira spp.* cyanobacteria or *Nannochloropsis* algae **90** (e.g., *N. oculata, N. australis, N. gaditana, N. granulate, N. limnetica, N. oceanica, N. salina, Nitzschia paleacea, Phaeodactylum tricornutum, Pavlova lutheri, Rebecca salina,* any strains or combinations thereof, or equivalent algae), and further comprising (i) an illumination system **120** that comprises multiple intense light sources **125,** e.g., light emitting diodes (LEDs, e.g., at between 400-700nm, within sub-ranges thereof, e.g., 400-500nm, 500-600nm, 600-700nm, and/or at specific wavelengths e.g., 650nm) that may be arranged in one or more horizontal and/or vertical panels (illustrated schematically and only in part), and (ii) a bubbling system **110** associated with a gas supply (e.g., air or nitrogen enriched with CO₂, e.g., including a CO₂ concentration of 30% or higher), which comprises multiple spargers of at least two types - sparger(s) **110A** with large nozzles (e.g., >1mm in diameter, possibly within a range of 1-5mm or within subranges thereof) for generating large bubbles **115A** that move fast (e.g., 100min⁻¹, indicating the rate of cumulative bubble volume to container volume, possibly within a range of ±30%, of 50-150 min⁻¹ or within subranges thereof) through the algae culture and mix it, and sparger(s) **110B** with small nozzles (e.g., <1mm in diameter, possibly within a range of 0.1-1mm or within subranges thereof) for generating small bubbles **115B** that move slowly (e.g., 5min⁻¹, indicating the rate of cumulative bubble volume to container volume, possibly within a range of ±30%, of 2-30 min⁻¹ or within subranges thereof) through the algae culture and enable CO₂ to diffuse to the algal cells. Possibly more than two types of nozzles may be used to control the mixing of the algae culture and the delivery of CO₂ at a sufficient concentration to the algae. The two or more types of spargers (each with multiple nozzles) may be distributed at one or more locations and may be configured to generate turbulent mixing of the algae in the cultivation container and provide CO₂ to the algae to the extent that maximizes or optimizes their growth and/or their content and/or composition, e.g., maximize the content and/or composition of bioavailable B12 (e.g., methylcobalamin or 5-deoxyadenosylcobalamin) and/or enhances the bioavailability of B12.

As illustrated schematically in the enlarged region of **Figure 4A****,** the very dense algae culture (e.g., having an algal density of at least 5 g/l, possibly within a range of 5-15 g/l or within subranges thereof) and localized intense light sources **125** create illuminated zones and dark zones within bioreactor **100,** and the intense agitation of the algae culture by large bubbles **115B** continuously mixes the liquid and moves algal cells **90** between the dark and the illuminated zones. In non-limiting examples, light sources **125** may reach an illumination intensity of any of at least 700 micromole-m⁻² s⁻¹, at least 1000 micromole-m⁻²s⁻¹, at least 1200 micromole·m⁻²s⁻¹, or intermediate values each, e.g., using at least 24 LEDs over an area of 4m² with a light path of about 2.5cm - defining the reach of the illuminated zones). The intense non-homogenous illumination, dark zone periods, and high level of CO₂ fed to algal cells **90** by small bubbles **115A** yield a high growth rate of the algae, and was also found to modify the biosynthetic pathways employed by the algae to form organic compound. For example, at high density conditions, the illuminated zones may extend to a few millimeters (e.g., 1-5mm) from point sources **125,** while the dark zones between the illuminated zones may extend over a few tens of millimeter (e.g., 20-30mm) between consecutive illuminated zones, so that individual algae cells spend periods in dark zones to assimilate CO₂ using the light energy absorbed in the illuminated zones.

The growing conditions may be monitored to maintain optimal growth, e.g., the temperature may be kept constant (e.g., at any of 15°C, 20°C, 25°C, 27°C, between any of 12-17°C, 15-20°C, 20-25°C, 25-30°C, 29-33°C, 30-35°C, 20-40°C, 20-50°C or within subranges thereof for *Nannochloropsis spp.,* or *Arthrospira spp.* cyanobacteria, depending on the specific type of algae) or be allowed to change within a specified range, chemical conditions such as the pH, O₂ and CO₂ content and/or content of various ions or compounds may be kept constant or be allowed to change within a specified range (e.g., between pH values of 6.7 and 7.2, or any subranges thereof). For example, the organic carbon content may be maintained at or around 20wt%. Alternatively or complementarily, CO₂ concentration may be kept at or below 40%, and/or the pH may be monitored to indicate and regulate the CO₂ concentration. Algae density may likewise be kept constant or be allowed to change within a specified range. The flow rates of small bubbles **115B** from sparger(s) **110B** with the small nozzles may be adjusted to increase or reduce CO₂ levels (e.g., increased above 5min⁻¹, e.g., to 7-10min⁻¹ to increase CO₂ levels, or reduced to 2min⁻¹ to reduce CO₂ levels). In certain embodiments, the level of various nutrients may be monitored, and additional nutrients may be provided via one or both bubble streams, e.g., phosphorous may be added to the gas supply if a low P level is detected or if growth is inhibited.

Algal slurry may be periodically or continuously removed from bioreactor **101,** e.g., to balance the growth of biomass, as indicated schematically in **Figure 4A****.** Disclosed algal products may comprise a botanical extract **140** that may be produced by mixing the algal slurry with ethanol (stage **130**) and then extracted from the mixture, e.g., by one or more stages of phase separation (stage **132**) and thermal separation (stage **134**). Disclosed algal products may comprise one or more of the separated phases. Non-limiting examples for parameters of the separation process include a volume ratio of at least 5:1 (or possible within the range 3.5:1 to 7:1 or intermediate subranges) between ethanol and the algal slurry (the mixture may be stirred for between 0.5-4 hours, e.g., for 2 hours), use of centrifugation for the phase separation, possibly under vacuum (e.g., under a pressure between 75-125 mbar) and a temperature range of between 55°C-65°C for the thermal separation. In certain embodiments, up to 90% of the algal mass may be removed per day.

In particular, the inventors have surprisingly found that extract **140** yielded by separation stages **132, 134** comprises more bioavailable B12 than pseudo-B12 (e.g., at least two or three times more bioavailable B12 than pseudo-B12). Accordingly, botanical extract **140** may be kept in its relatively pristine form, avoiding high temperatures and chemical modification.

As additionally illustrated schematically in **Figures 3B-3D,** cultivation system **101** comprises at least one first sparging unit **110A** having a plurality of nozzles and configured to distribute a first predetermined fluid **115A** (e.g., air and/or nitrogen bubbles) into a water-filled algae cultivation container **100** (e.g., a bio-reactor) at a first operating flow rate so as to allow mixing therein (indicated schematically by arrows **118**). Cultivation system **101** may further comprise at least one second sparging unit **110B** having a plurality of nozzles and configured to distribute a second predetermined fluid **115B** (e.g., gas bubbles with CO₂, indicated schematically, and/or dissolved phosphorus for mass transfer) into container **100** at a second operating flow rate. Fluids exiting container **100,** such as gas from second predetermined fluid **115B** may be recycled **113** to fully utilize remaining CO₂ therein (illustrated schematically).

Cultivation system **101** may further comprise at least one controller **103** in communication with sparging units **110** and configured to control the first operating flow rate and the second operating flow rate provided thereby. Controller **103** may comprise one or more processing units that implement computer code. For example, at least one nozzle of first sparging unit **110A** and/or at least one nozzle of second sparging unit **110B** may be configured to distribute fluid into cultivation container **100** based on instructions from at least one controller **103.** In some embodiments, the first operating flow rate may be based on the second operating flow rate and/or at least one of the operating flow rates may be predetermined. In some embodiments, the first operating flow rate may be adapted to allow turbulent mixing of the algae in cultivation container **100.** In some embodiments, the second operating flow rate may be adapted to allow mass transfer and/or assimilation of materials in a liquid in cultivation container **100.** Information may flow between controller **103** and sparging units **110,** as well as between controller **103** and other elements in the system, as indicated schematically by the arrows.

Second predetermined fluid **115B** may include gas bubbles with over 30% CO₂ concentration. The source for the first predetermined fluid(s) and/or for the second predetermined fluid(s) may be external to cultivation system **101,** for example geothermal power stations may provide a source of dissolved carbon and/or sulfur for the second predetermined fluid.

The first operating flow rate of at least one nozzle of first sparging unit **110A** (e.g., 100 ml/min) may be different from the second operating flow rate of at least one nozzle of second sparging unit **110B** (e.g., 5 ml/min). In some embodiments, at least one nozzle of first sparging unit **110A** may have a diameter larger than about 1 millimeter. In some embodiments, at least one nozzle of second sparging unit **110B** may have a diameter smaller than about 1 millimeter. In some embodiments, nozzles of first sparging unit **110A** as well as of second sparging unit **110B** may distribute the same fluid (e.g., air), with nozzles of each sparging unit having different diameters. Larger apertures of first sparging unit **110A** may be configured to provide first predetermined fluid **115A** with large bubbles (e.g., of air and/or nitrogen) to agitate and mix **118** the suspended biomass in container **100,** while smaller apertures of second sparging unit **110B** may be configured to provide second predetermined fluid **115B** with small bubbles (e.g., of or comprising CO₂) to transfer CO₂ from the gas to the liquid to be accessible to the suspended biomass in container **100.** Advantageously, the difference in the size of the delivered bubbles may prevent the combination of the bubbles of streams **115A, 115B,** providing simultaneously mixing **118** by the high throughput of big and fast bubbles in stream **115A** and effective CO₂ supply by the small throughput of small and slow bubbles in stream **115B.**

Cultivation system **101** may further include a physical barrier **104** configured to separate the first fluid distributed by first sparging unit **110A** and the second fluid distributed by second sparging unit **110B** within cultivation container **100.** In some embodiments, at least one nozzle of first sparging unit **110A** and/or of second sparging unit **110B** may be embedded into physical barrier **104** (not shown). In some embodiments, physical barrier **104** may be adapted to allow flow from one side of barrier **104** (having a first fluid distribution) to the other side of barrier **104** (having a second fluid distribution) at predefined (e.g., upper and lower) locations of cultivation container **100,** in order to create a controlled flow within the container **100.**

Cultivation container **100** with physical barrier **104** may include at least one light source **125** embedded into the physical barrier **104** such that container **100** may be illuminated (illumination denoted schematically by arrows **203**) from within by from at least one light source **125** embedded into the physical barrier **104.** Cultivation container **100** may include a plurality of physical barriers **104,** each including at least one light source **125,** such that a modular system may be created with algae and/or cyanobacteria growing between adjacent physical barriers **104,** wherein at least one controller **103** may control illumination for all light sources **125** embedded into the physical barriers **104.** In some embodiments, physical barriers **104** may comprise illumination system **120** with multiple intense light sources **125.**

In certain embodiments, cultivation system **101** may be configured to reach a very high density of the cultivated biomass with a corresponding small optical depth that yield a relatively thin illuminated zone **116** and a much thicker dark zone **117** in container **100,** with the biomass being continuously agitated **118** (e.g., by strong bubbling of fluids **115A** and optionally **115B**) so that individual cells of algae and/or cyanobacteria have but a brief residence time in illuminated zone **116** before returning to dark zone **117.** In non-limiting examples, the thickness of illuminated zone **116** may be configured to be between 0.1cm and 1.5cm, depending on the density of the suspension and the illumination density, and may be controlled by controller **103** and adjusted according to specified requirements. Accordingly, illumination **203** (and particularly UV components thereof) may be set at very high levels as the brief residence time prevents illumination damage to the individual cells.

Cultivation system **101** may further include at least one sensor **105** (e.g., a temperature sensor) coupled to controller **103** and configured to detect at least one feature within cultivation container **100.** For example, at least one sensor **105** may be configured to detect any of the pH levels, the temperature, and the pressure conditions within cultivation container **100** and/or sections thereof. In some embodiments, at least one sensor **105** may also be configured to detect parameters external to cultivation container **100,** for example measuring mass flow of the gas emissions from cultivation container **100** to determine an amount of substance that was absorbed in the algae cells by subtracting the emitted amount from the amount inserted into the container (e.g., by second sparging unit **102**).

Cultivation system **101** may further include at least one database **106** (and/or memory unit) configured to store algorithms for operation of controller **103,** for instance database of operating rates for each nozzle and/or each sparging unit. In some embodiments, cultivation system **101** may further include a power source **107** coupled to controller **103** and configured to provide electrical power to cultivation system **101.** Power source **107** may be configured to power at least one first sparging unit **110A** and at least one second sparging unit **110B,** e.g., to operate at different rates.

Data gathered by at least one sensor **105** may be analyzed by controller (or processor) **103** to detect if an attribute exceeds a predetermined threshold, for instance threshold for pH level and/or temperature and/or CO₂ concentration within the container **100.** In case conditions within cultivation container **100** (e.g., as detected by sensor **105**) exceed at least one threshold, then controller **103** may operate at least one nozzle of first sparging unit **110A** and/or at least one nozzle of second sparging unit **110B** at a different flow rate. For example, detecting CO₂ concentration within the container **100** exceeds 40% (or detecting low pH levels) may cause at least one nozzle of second sparging unit **110B** to lower flow rate of second sparging unit **110B** to about 2 millimeters/minute. In some embodiments, at least one nozzle of second sparging unit **110B** may operate only upon receiving a signal from sensor **105** that an attribute exceeds a predetermined threshold, and not operated in a constant rate.

At least one nozzle of first sparging unit **110A** may be configured to operate only upon receiving a signal from sensor **105** that an attribute exceeds a predetermined threshold, for example increasing mixing flow **118** as the density of algal population increases. At least one nozzle of first sparging unit **101** and/or at least one nozzle of second sparging unit **110B** may operate in a constant rate, continuously, or possibly intermittently. At least one nozzle of first sparging unit **110A** and/or at least one nozzle of second sparging unit **110B** may operate in a non-constant rate continuously, or possibly intermittently.

Cultivation container **100** may comprise a bubble column configuration with at least one first sparging unit **110A** and at least one second sparging unit **110B** positioned on the same surface of the bubble column container. Cultivation container 100 may have an airlift configuration with at least one second sparging unit **110B** positioned at a bottom portion of a downcomer that may be distal to sensor **105,** such that residence time of bubbles from at least one second sparging unit **110B** may be increased.

Cultivation system **101** may be configured to enable maintaining at least 20% organic carbon within container **100,** calculated in addition to carbon provided as CO₂ bubbles. In some embodiments, at least portion(s) of the algae within container **100** may comprise any photosynthetic microorganism such as algae and/or cyanobacteria used to prepare spirulina preparations, including, e.g., *Arthrospira platensis, A. fusiformis* and/or *A. maxima* as well as *Nannochloropsis spp.*

In some embodiments, optimized controlled provision of the harmful UV radiation may allow increasing the amounts and/or bioavailability of B12 (e.g., methylcobalamin and/or 5-deoxyadenosylcobalamin) and/or increase the content of PheLys, in the cultivated algae and on the extracted spirulina while avoiding damaging the growing algae or the growing rate. In some embodiments, the on/off nature of the radiation provision may allow controlling the amount of harmful radiation provided. Moreover, the constant mixing and/or bubbling **118** of the suspension in container (by sparging units **110**) ensures only brief exposures of any individual algal or cyanobacterial cells to the intense radiation, preventing photoinhibition and damage to the cells. Controller **103** may be respectively configured to control the extent of turbulence provided by sparging units **110** to avoid radiation damage to the cells. For example, method **300** and cultivation system **101** may be configured to achieve the required UV photo-modulation in a thin-film cultivation system, by turning the UV light source on and off and/or by creating shade patterns that yield intermittent illumination of the algae and/or cyanobacteria. In bubbled cultivation systems **101,** the relative velocities of the flows of the culture suspension and of the gas bubbles relative to the UV light source may be controlled to achieve specified patterns of on/off UV exposure cycles.

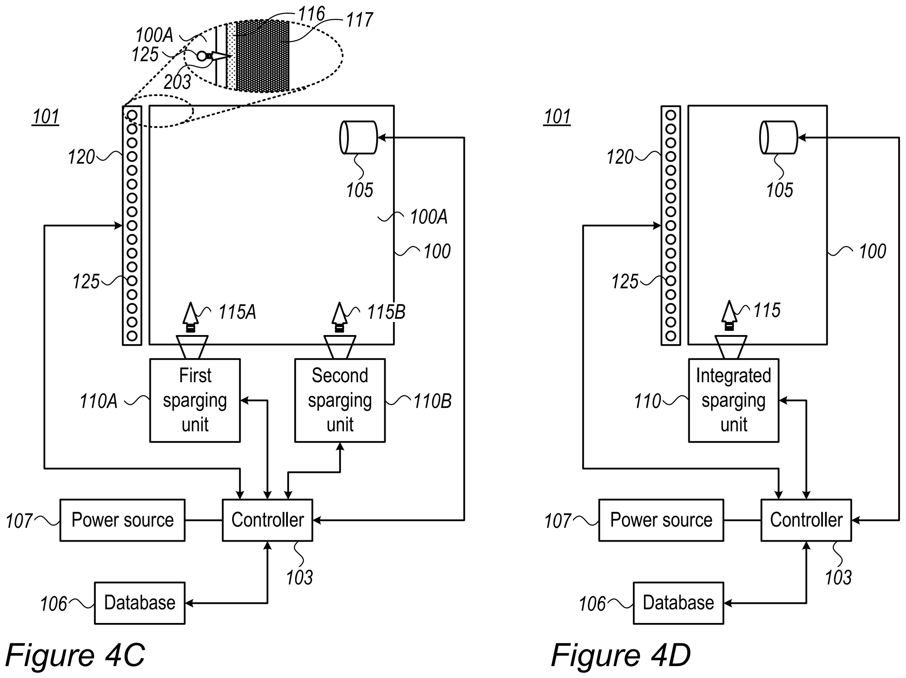

**Figure 4C** and **4D** illustrates schematically embodiments of cultivation systems **101,** according to some embodiments of the invention. Cultivation system **101** may comprise at least one illumination unit **120** coupled to controller **103,** to illuminate cultivation container **100.** Illumination unit(s) **120** and controller **103** (or another controller) may be included in a bioreactor illumination system for growing algae and/or cyanobacteria. The distance between cultivation container **100** and illumination unit(s) **120** may be modified to control the illumination received by cultivation container **100.** For example, bringing illumination unit(s) **120** closer to cultivation container **100** increases illumination of the culture therein. The distance between cultivation container **100** and illumination unit(s) **120** may be controlled by controller **103,** for example, included in the illumination system. In addition to, or instead of, changing the distance of illumination unit **120** from cultivation container **100,** the illumination intensity of light sources **125** in illumination unit **120** may be controlled. Illumination unit **120** may include at least one light source **125** (e.g., LED), and each light source **125** may be controlled separately by controller **103.** In some embodiments, one or more light source(s) **125** may be controlled to illuminate with a different intensity than another light source(s) **125.** All light sources **125** may be controlled to change the illumination intensity, either manually or according to preset timing and/or sensed conditions in cultivation container **100.** At least some of light sources **125** may be configured to emit light in the UV spectrum, for example, in both the UVA and the UVB range. The ratio between the emitted radiation in UVA and UVB (UVA/UVB ratio) may be between 10 and 15, e.g., 10, 12, 14, 15 UBA/UVB or have intermediate values. The UV radiation may be provided at 1,000-10,000 kJ/m², for example, 2000 kJ/m², 5000 kJ/m², 7000 kJ/m², 9000 kJ/m² or any other intermediate value. Controller **103** may be configured to control the provision of the UV radiation using radiation pulses, e.g., each pulse may last less than 0.01 sec, e.g., 0.008, 0.009, 0.0095 or 0.0099 seconds, or any intermediate value, and between 1-100 of these pulses may be provided per second, for example, 10 times per second. It is noted that approximately 0.01sec of 1,000kJ/m² illumination yields about ten times the intensity of solar UV radiation, which changes the chemical composition of the algae and/or cyanobacteria to yield extract compositions disclosed below. Some light sources **125** may be configured to emit light at the visible spectrum (e.g., wavelength of 400-700 nm). In some embodiments, some algae, e.g., *Nannochloropsis spp.* may be grown under solar illumination, as long as the algal extracts include more bioavailable B12 than pseudo-B12 and/or increased content of PheLys.

The amount of light delivered to cultivation container **100** may be defined as an average of light flux delivered to the surface of cultivation container **100.** Cultivation system **101** may be used to support ultra-high-density cultures (e.g., having a biomass density of 1, 5 or up to 10 g/l, or intermediate values), with illumination unit(s) **120** configured to have a light distribution of light source(s) **125** that provides an average light flux per algae/cyanobacteria cell that is comparable to average light flux per cell of low-density cultures achieving a similar level of average illumination per cell. Light intensity within cultivation container **100** may be measured with at least one sensor **105** and adjusted by controller **103.** For example, for ultra-high-density cultures a typical thickness of illuminated zone **116** may range, e.g., between 1mm and 5mm, while a typical thickness of dark zone **117** may range, e.g., between 20mm and 30mm). The distance of illumination unit 120 from the side of container **100** may be adjusted with respect to the prevailing optical thickness (or optical depth, OD) of the biomass suspension to avoid photoinhibition and/or photo-bleaching. For example, at initial cultivation stages, when the culture density is relatively low, illumination unit **120** may be initially kept at a distance from the side of container **100** to biomass growth, while at later cultivation stages, once the OD increases, illumination unit **120** may be brought closer to the side of container **100** to promote biomass growth.

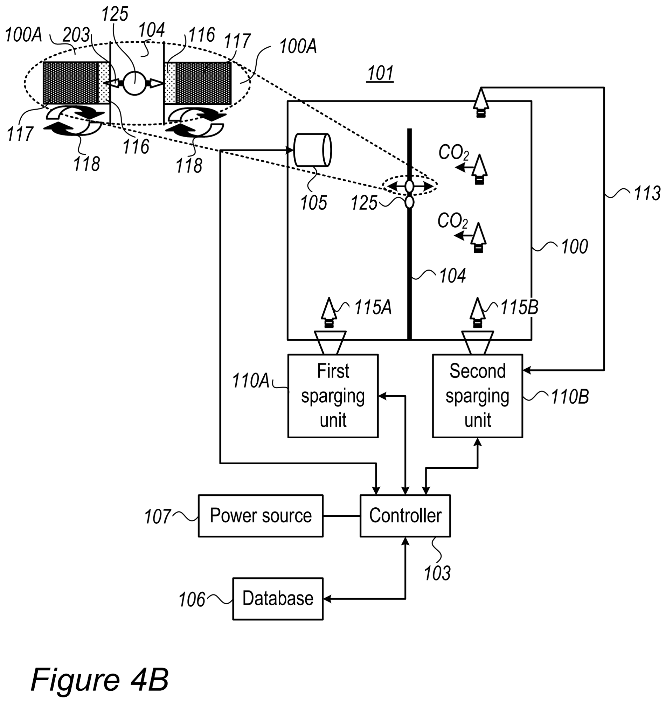

Ultra-high-density cultures be continuously and/or intermittently mixed and/or agitated (indicated schematically by arrows **118** in **Figure 4B**), by mechanical means and/or by strong bubbling of fluids **115A** and optionally **115B,** to cause the algae and/or cyanobacteria to move between illuminated zone **116** and dark zone **117** and to prevent damage to the cells, yielding illumination cycles for the algae/cyanobacteria (between illuminated zone **116** and dark zone **117**) due to the short light passage. Ultra-high-density cultures may be illuminated with various wavelengths, since in such biomass densities the wavelength may have nearly no effect on the growth due to the short light passage. This is in contrast to the common practice, according to which algae are illuminated with specific wavelengths (e.g., with blue light) for normal growth since algae should respond to light differently. However, the inventors have found out experimentally that illumination with any wavelength may be used for ultra-high-density cultures.

The light penetration into cultivation container **100** may correspond to at least one of the light intensity, the light wavelength, the specific algal strain, and/or the algal culture density. It should be noted that the light penetration into cultivation container **110** may determine the volumetric ratio between illuminated zones **116** and dark zones **117** within cultivation container **110,** and thus may affect the light intensity provided by illumination units **120,** the gas flow rate through first sparging unit **110A,** the gas flow rate through second sparging unit **110B,** etc., which may be adjusted and optimized respectively.

In some embodiments, cultivation container **100** may be illuminated by illumination unit(s) **120** to provide a daily amount of over 90% of maximal algae growth within the cultivation container **100.** In some embodiments, illumination unit(s) **201** may be configured to illuminate the suspension in container **100** in a non-homogenous manner, e.g., using few high intensity light sources **120** spaced apart, because the illumination of the cells is local and temporally controlled (through agitation **118**). The inventors have found out that high intensity intermittent illumination actually enhances growth of algae and/or cyanobacteria, over common practice configurations with homogenous distribution of low intensity light sources.

For example, the illumination photon flux density of at least one light source **120** may be 1200 µmole/m²s. In various embodiments, the photon flux density may range between 1000-1500 µmole/m²s or have intermediate values. In some embodiments, illumination unit(s) **120** may include at least four light sources 125 per m². As an illustrative non-limiting example, illumination unit **120** having a surface area of about 6m², a light path (thickness of illuminated zone **116**) of about 4cm may include 24 LED light sources **125,** each having light flux of 1200 µmole/m²s. In some embodiments, at least a portion of the cyanobacteria within container **100** comprises *Arthrospira spp.* used to prepare spirulina extracts and/or at least a portion of the green algae within container **100** comprises *Nannochloropsis spp.* used to prepare algal extracts.

Controller **103** may be configured to control the illumination wavelength of the at least one light source **125,** for instance with a dedicated illumination module adapted to modify the wavelength of the emitted illumination. In some embodiments, a constant temperature of 27°C may be maintained within the container 100. In some embodiments, controller **103** may be configured to control at least one light source **125** to illuminate with a wavelength of 650 nanometers. It should be noted that, according to common practice, algae are illuminated with a particular wavelength (e.g., with blue light) for optimal growth, but experiments conducted by the inventors have shown that illumination with other wavelengths (e.g., with red light) may be used for enhanced growth.

As illustrated schematically in **Figure 4D****,** cultivation system **101** may be configured to operate with an integrated sparging unit **110,** which may comprise nozzles of two or more types, integrating sparging units **110A, 110B.** Integrated sparging unit **110** may be configured to distribute a predetermined fluid **115** into cultivation container **100.** Predetermined fluid **115** may comprise one or both of predetermined fluids **115A, 115B,** or various mixtures of parts thereof. For example, integrated sparging unit **110** may include at least one nozzle to distribute first predetermined fluid **115A** and at least one nozzle (e.g., having a different diameter) to distribute second predetermined fluid **115B,** which in combination are denoted schematically in a non-limiting manner as predetermined fluid **115.** Sparging unit **110** may be configured to generate turbulent mixing of the algae and/or cyanobacteria in cultivation container **100,** as well as provide CO₂ for assimilation thereby, e.g., as disclosed with reference to **Figures 3B** and **3C.** In certain embodiments, cultivation systems **101** may comprise combinations of dedicated sparging units **110A, 110B** and integrated sparging unit(s) **110.**

The inventors have found out that disclosed algal extracts, extracted from algae (or cyanobacteria) grown under the conditions disclosed above, contain bioavailable, metabolically active B12 (e.g., methylcobalamin and/or 5-deoxyadenosylcobalamin,), for example, more bioavailable B12 than pseudo-B12. The extract, biomass, or portions thereof may be used as biostimulant compositions for enhancing soil content of organic carbon, providing a significant portion of B12 through bioavailable methylcobalamin B12.

A long-term study has shown that the content of bioavailable (e.g., bioavailable) MeB12 under the disclosed growing conditions was consistent and provided a reliable source for bioavailable MeB12. **Table 2** provides results for bioavailable MeB12 and pseudo-B12 in disclosed spirulina biomass tracked over nine months. These results were consistent also over six independent repetitions, yielding similar results. Although both pseudo- and active forms of vitamin B₁₂ were observed, the active form largely dominates the composition (>98%). While the composition of traditional, naturally occurring spirulina is dominated by pseudo-vitamin B12, with a net-negative content of active B12, the results obtained here illustrate the effects of photonic management on enhancing natural metabolic pathways for achieving active, and net-active B₁₂ in Spirulina.

**Table 2: Bioavailable MeB12 and pseudo-B 12 in disclosed spirulina biomass.**

| | Month #3 | | Month #9 | |
|---|---|---|---|---|
| | µg /100 g | ± SD | µg /100 g | ± SD |
| Pseudo-Vitamin B12 | 0.02 | 6% | 0.04 | 6% |
| Active Vitamin B12 | 1.7 | 4% | 1.7 | 5% |
| Net Active Vitamin B12 | 1.7 | 4% | 1.6 | 5% |

Accordingly, bioavailable active vitamin B12 content was found to be statistically consistent over nine months of continued cultivation, yielding a concentration of 1.64 µg/100g. Moreover, disclosed spirulina products also include macro- and micro-nutrients (e.g., essential amino acids, alpha-linolenic acid (ω-3 fatty acid) and linoleic acid (ω-6 fatty acid), various minerals, including calcium, potassium, magnesium and iron, and vitamins, such as beta-carotene) and therefore provide good products for enhancing soil organic carbon content, in addition to the synergistic effects with amino acids Phe and Lys described herein. It is further noted that due to the negative carbon footprint of algae grown as disclosed, the corresponding biostimulant compositions may also have a negative, or at least reduced carbon footprint, in addition to the disclosed synergistic effects. Hence, incorporating algal extracts as biostimulants may contribute to direct decarbonization of agricultural soil treatments by incorporating algae from the geothermal park in soil treatments and indirect decarbonization of agricultural soil treatments by means of issuing, selling, and purchasing carbon credits between enterprises.

In the above description, an embodiment is an example or implementation of the invention. The various appearances of "one embodiment", "an embodiment", "certain embodiments" or "some embodiments" do not necessarily all refer to the same embodiments. Although various features of the invention may be described in the context of a single embodiment, the features may also be provided separately or in any suitable combination. Conversely, although the invention may be described herein in the context of separate embodiments for clarity, the invention may also be implemented in a single embodiment. Certain embodiments of the invention may include features from different embodiments disclosed above, and certain embodiments may incorporate elements from other embodiments disclosed above. The disclosure of elements of the invention in the context of a specific embodiment is not to be taken as limiting their use in the specific embodiment alone. Furthermore, it is to be understood that the invention can be carried out or practiced in various ways and that the invention can be implemented in certain embodiments other than the ones outlined in the description above.

The invention is not limited to those diagrams or to the corresponding descriptions. For example, flow need not move through each illustrated box or state, or in exactly the same order as illustrated and described. Meanings of technical and scientific terms used herein are to be commonly understood as by one of ordinary skill in the art to which the invention belongs, unless otherwise defined. While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention should not be limited by what has thus far been described, but by the appended claims and their legal equivalents.

## Claims

1. A biostimulant composition comprising:
bioavailable B12, and
PheLys comprising phenylalanine (Phe) and lysine (Lys),
wherein upon application the biostimulant composition enhances soil organic carbon.

2. The biostimulant composition of claim 1, wherein the bioavailable B12 and the PheLys are in dry powder form, and are mixed in water to form a biostimulant solution having between 0.2-0.5 mg/l of bioavailable B12 and between 5-15 g/l of PheLys.

3. The biostimulant composition of claim 1 or 2, having a ratio between bioavailable B12 and PheLys that yields an application of between 50-80 g/ha of Phe and Lys and between 1-5 mg/ha of bioavailable B12.

4. The biostimulant composition of any one of claims 1-3, wherein the bioavailable B12 and the PheLys are extracted from spirulina, including *Arthrospira* spp. that was cultivated under photosynthetically controlled conditions to yield upregulated bio-active compounds comprising bioavailable B12 that is more bioavailable than pseudo-B12 and increased content of PheLys, wherein the photosynthetically controlled conditions comprise cultivating the *Arthrospira spp.* in a ultra-high-density culture having a density between 3g/l and 10g/l, under 31±2°C, at pH of 10.8 ± 0.2 and under irradiance of between 700 and 1500 µmol/(m²s) with an ultraviolet radiation intensity of between 70-150 µmol/m²s; and optionally the bioavailable B12 and the PheLys are made of a water-based extract that is produced by water-extraction and cycles of freezing and thawing applied to the cultivated *Arthrospira spp.*

5. The biostimulant composition of any one of claims 1-3, wherein the bioavailable B12 and the PheLys are extracted from *Nannochloropsis spp.* that was cultivated under photosynthetically controlled conditions to yield upregulated bio-active compounds comprising bioavailable B12 that is more bioavailable than pseudo-B12 and increased content of PheLys, wherein the photosynthetically controlled conditions comprise cultivating the *Nannochloropsis spp.* in a ultra-high-density culture having a density between 3g/l and 10g/l, under 15-25°C, at pH of 10.8 ± 0.2 and under irradiance of between 700 and 1500 µmol/(m²s) and an ultraviolet radiation intensity of between 70-150 µmol/m²s ; and optionally the bioavailable B12 and the PheLys are made of a water-based extract that is produced by water-extraction and cycles of freezing and thawing applied to the cultivated *Nannochloropsis spp.*

6. The biostimulant composition of any one of claims 1-3, wherein the bioavailable B12 and the PheLys are derived from cultivated yeast and/or cultivated bacteria.

7. A dry mass extract of cultivated *Arthrospira spp.* that includes more than 10 g Phe per 100 g dry biomass, more than 10 g Lys per 100 g dry biomass and at least twice bioavailable B12 compared pseudo B12.

8. A method comprising enhancing soil organic carbon by applying a mixture of bioavailable B12 and PheLys comprising phenylalanine (Phe) and lysine (Lys).

9. The method of claim 8, further comprising mixing the bioavailable B12 and the PheLys in dry powder form with water to form a biostimulant solution comprising thereof, and applying the mixture by irrigation.

10. The method of claim 9, wherein, in the biostimulant solution, a concentration of the bioavailable B12 is between 0.2-0.5 mg/l and a concentration of the PheLys is between 5-15 g/l.

11. The method of claim 8, wherein the mixture has a ratio between bioavailable B12 and PheLys that yields an application of between 50-80 g/ha of Phe and Lys and between 1-5 mg/ha of bioavailable B12.

12. The method of any one of claims 8-11, wherein the bioavailable B12 and the PheLys is extracted from a spirulina extract, made of *Arthrospira spp.* that was cultivated under photosynthetically controlled conditions to yield upregulated bio-active compounds comprising bioavailable B12 that is more bioavailable than pseudo-B12 and increased content of PheLys, the method further comprising cultivating the *Arthrospira spp.* under the photosynthetically controlled conditions that comprise a ultra-high-density culture having a density between 3g/l and 10g/l, a temperature of 31±2°C, a pH of 10.8 ± 0.2 and irradiance of between 700 and 1500 µmol/(m²s) with an ultraviolet radiation intensity of between 70-150 µmol/m²s; and optionally further comprising preparing the bioavailable B12 and the PheLys from a water-based extract that is produced by water-extraction and cycles of freezing and thawing applied to the cultivated *Arthrospira spp.*; and optionally further comprising preparing the bioavailable B12 and the PheLys from at least one of: the wet or dried spirulina biomass, spirulina paste made of the spirulina biomass, spirulina powder and/or spirulina liquid made of the spirulina biomass.

13. The method of any one of claims 8-11, wherein the bioavailable B12 and the PheLys are extracted from a spirulina extract, made of *Nannochloropsis spp.* that was cultivated under photosynthetically controlled conditions to yield upregulated bio-active compounds comprising bioavailable B12 that is more bioavailable than pseudo-B12 and increased content of PheLys, the method further comprising cultivating the *Arthrospira spp.* under the photosynthetically controlled conditions that comprise a ultra-high-density culture having a density between 3g/l and 10g/l, a temperature between 15-25°C, a pH of 10.8 ± 0.2 and under irradiance of between 700 and 1500 µmol/(m²s) with ultraviolet radiation intensity of between 70-150 µmol/m²s; and optionally further comprising preparing the bioavailable B12 and the PheLys from a water-based extract that is produced by water-extraction and cycles of freezing and thawing applied to the cultivated *Nannochloropsis spp*.; and optionally further comprising preparing the bioavailable B12 and the PheLys from at least one of: the wet or dried *Nannochloropsis* biomass, *Nannochloropsis* paste made of the *Nannochloropsis* biomass, *Nannochloropsis* powder and/or *Nannochloropsis* liquid made of the *Nannochloropsis* biomass.

14. The method of any one of claims 8-11, further comprising deriving the bioavailable B12 and the PheLys are derived from cultivated yeast and/or cultivated bacteria.
